# EUROPEAN PATENT APPLICATION

(11) **EP 2 860 525 A1**
(43) Date of publication of application: **15.04.2015**
(21) Application number: 13187966.0
(22) Date of filing: 09.10.2013
(51) Int. Cl.: G01N 33/68

(54) **ProSP-B for interstitial lung diseases**

(71) Applicant: Roche Diagnostics GmbH, 68305 Mannheim (DE); F. Hoffmann-La Roche AG, 4070 Basel (CH)
(72) Inventor: Klemt, Volker, 82362 Weilheim (DE); Roeddiger, Ralf, 69517 Gorxheimertal (DE); Pfeffer, Michael, 82377 Penzberg (DE); Boehm, Christine, 6331 Huenenberg (CH); Ehret, Christoph, 81477 München (DE)
(74) Representative: Dick, Alexander

(57) **Abstract**

The present invention is directed to a method of monitoring interstitial lung disease in a patient having interstitial lung disease. The method is based on measuring the level of C-terminal proSP-B or proSP-B in a first and second sample from a patient. Further envisaged is a method of assessing whether a patient suffering from interstitial lung disease responds to a therapy against interstitial lung disease. Moreover, the present invention contemplates a method for prediction whether a patient having interstitial lung disease as likely to show regression of interstitial lung disease.

## Description

The present invention is directed to a method of monitoring interstitial lung disease in a patient having interstitial lung disease. The method is based on measuring the level of C-terminal proSP-B or proSP-B in a first and second sample from a patient. Further envisaged is a method of assessing whether a patient suffering from interstitial lung disease responds to a therapy against interstitial lung disease. Moreover, the present invention contemplates a method for prediction whether a patient having interstitial lung disease as likely to show regression or progression of interstitial lung disease.

Interstitial lung diseases (ILDs) are characterized by progressive shortness of breath which starts at an early disease stage during physical exercise and deteriorates towards shortness at rest and nonproductive cough when the disease is more advanced.

The shortness of breath results from damage to the lining of the alveoli leading to inflammation and fibrosis of the interstitium. The cause for the alveoli damage varies and more than 300 different entities if interstitial lung diseases are known. External factors that can contribute to the disease include organic or inorganic material, drug reactions/toxicity and infections. There is, however, a large group of IDLs of unknown etiology ("idiopathic interstitial pneumonias (IIP")) and the most common ones are sarcoidosis and idiopathic pulmonary fibrosis (IPF). An overview on the classification of interstitial lung disease is given in by Wells et al, Eur Respir Rev 2013; 22: 128, 158-162, see in particular Fig. 1.

There is no curative treatment available so far. Antiinflammatory, antifibrotic, antioxidant and immunomodulatory drugs might be used for the IIPs albeit for none of the single drugs or drug combinations sufficient evidence is available. Lung transplantation might be applied in selected severe cases. Pirfenidone is a recently launched drug and can be used for the mild to moderate IPF to slow down disease progression, but definitive evidence is pending. The compound has pleiotropic, antiinflammatory, antifibrotic, and antioxidant properties, with antagonism of TGF-β1 effects.

Idiopathic pulmonary fibrosis (IPF) is defined as a specific form of chronic progressively fibrosing IIP of unknown cause that occurs primarily in relatively elderly adults. It is the most pernicious and frequent of the ILDs, accounting for 55% of IIP. The prevalence of IPF in the European Union is 120 000 and an estimated 40 000 new cases are diagnosed each year. (Ragu G et al. Am J Respir Crit Care Med (2011) 183, 788-824).

IPF is associated with a characteristic radiological and/ or histopathological pattern of usual interstitial pneumonia (UIP). In an 2011 guideline update the American Thoracic Society, the European Respiratory Society, the Japanese Respiratory Society, and the Latin American Thoracic Association have revisited the diagnostic criteria of IPF which are based on high resolution computed tomography (HRCT) scan and surgical lung biopsy (SLB) and defined the criteria for define, probable, possible and unlikely diagnosis of IPF. (Ragu G et al. Am J Respir Crit Care Med (2011) 183, 788-824).

Imaging criteria for the diagnosis of a UIP pattern on HRCT include the presence of bilateral, predominantly subpleural, basal reticular abnormalities and the absence of additional features considered incompatible with a diagnosis of IPF. The definite diagnosis requires the presence of honeycombing which present as clustered cystic air space, usually of comparable diameters (3-10 mm, occasionally larger), with well-defined thickened walls and mainly subpleural. If diagnosis cannot be achieved by HRCT, surgical lung biopsy might be applied.

Frequently IPF presents as a slow, gradual decline in pulmonary function over many years and eventual death from respiratory failure or complicating comorbidity. However, some patients may experience an accelerated decline and others episodes of acute respiratory worsening (see in particular Figure 4 of Raghu G et al. Am J Respir Crit Care Med (2011) 183, 788-824). Comorbidities may impact the disease course as well.

The reason for the different progression patterns are unknown and it is unclear if such patterns represent distinct phenotypes of IPF or if they are influenced by factors like environment, geographic, ethnic, or others (Ragu G et al. Am J Respir Crit Care Med (2011) 183, 788-824).

Disease progression is commonly characterized on resting pulmonary function test measurements and/or extent of radiologic abnormalities. However, it is unknown if these staging approaches are relevant to clinical decision making.

Criteria for acute exacerbation of IPF, which can occur any time in the disease course, have historically included an unexplained worsening of dyspnea within 1 month, evidence of hypoxemia as defined by worsened or severely impaired gas exchange, new radiographic alveolar infiltrates, and an absence of an alternative explanation such as infection, pulmonary embolism, pneumothorax, or heart failure. Clinically worsened cough, fever, and/or increased sputum can be observed. (Ragu G et al. Am J Respir Crit Care Med (2011) 183, 788-824)

It is important to identify those IPF patients with increased risk for mortality and consider them early for lung transplantation. However, there are currently no characteristics or markers which would identify early the fast disease progressors or even predict disease progression type. The 2011 guideline describes selected features which are commonly used in clinical practice and were observed as somehow associated with increased mortality in IPF, but are still lacking clear evidence (see in particular Figure 7 in Ragu G et al. Am J Respir Crit Care Med (2011) 183, 788-824).

Baseline pulmonary function test values like baseline FVC, for instance, might be affected by comorbidities like emphysema or obesity, technical differences and have unclear predictive value. Diffusing capacity for carbon monoxide (DLCO, single breath, hemoglobin corrected) seems to be a more reliable measure predictive of survival at baseline. A threshold of approximately 40 percent has been associated with an increased risk of mortality. (Ragu G et al. Am J Respir Crit Care Med Vol 183. pp 788-824, 2011 and references therein)

With respect to serum and bronchoalveolar lavage (BAL) few markers have been investigated and increased levels of some may correlate with increased mortality risk (Ragu G et al. Am J Respir Crit Care Med (2011) 183, 788-824 and references therein):
- KL-6 (Krebs von den Lungen-6)is produced by regenerating type II pneumocytes and serum levels of KL-6 have been shown to be elevated in patients with IPF, and these levels may correlate with increased risk of subsequent mortality.
- Serum levels of surfactant protein A and D are also elevated in patients with IPF and are predictive of survival Serum surfactant proteins- A and -D as biomarkers in idiopathic pulmonary fibrosis (see Greene KE, King TE Jr, Kuroki Y, Bucher-Bartelson B, Hunninghake GW, Newman LS, Nagae H, Mason RJ. Eur Respir J (2002) 19:439-446; Serum surfactant protein-A is a strong predictor of early mortality in idiopathic pulmonary fibrosis; or Kinder BW, Brown KK, McCormack FX, Ix JH, Kervitsky A, Schwarz MI, King TE Jr.,Chest (2009)135:1557-1563;Serum surfactant proteins A and D as prognostic factors in idiopathic pulmonary fibrosis and their relationship to disease extent, or Takahashi H et al.,Am J Respir Crit Care Med (2000) 162:1109-1114).
- A relationship between serum CCL18, other chemokines, and serum brain natiuret-ic peptide levels and mortality was observed.
- Studies of plasma and BAL matrix metalloproteinase (MMP) levels suggest that MMP1 and MMP7 are increased in patients with IPF, and MMP7 levels may correlate with disease severity.
- BAL levels of SP-A appear predictive of survival.
- Cellular analysis of BAL is of unclear predictive value in IPF.
- Preliminary evidence suggests that the presence of circulating fibrocytes (mesen-chymal progenitor cells) is associated with worse short-term survival (228).

Surfactant is synthesized and secreted into the alveolar fluid by type II pneumocytes and is composed of approximately 80% phospholipids, 10% neutral lipids, and 10% proteins.

There are four surfactant proteins, the hydrophilic water soluble surfactant proteins A and D (SP-A and SP-D) which are important for host defense, but have less impact than the hydrophobic surfactant proteins on the biophysical, and the hydrophobic surfactant proteins B and C which are critical for optimizing surface tension reduction.

The serum levels of the hydrophilic surfactant proteins A and D have been investigated in the past and increased levels of both surfactant proteins compared to healthy or patients with other lung diseases were observed. It was therefore concluded that both markers could serve as potential biomarkers in IPF. Longitudinal serial measurements, however, did not show significant changes with time (Greene 2002).

Takahashi et al. (Am J Respir Crit Care Med (2000) 162:1109-1114) showed in their investigation that SP-A and SP-D levels in patients who died within 3 years were significantly higher than in patients who were still alive after 3 years.

They also showed that both SP-A and SP-D concentrations were significantly correlate with the extent of alveolitis (a reversible change), whereas they did not correlate with the progression of fibrosis (an irreversible change). The SP-D concentration, unlike that of SPA, was also related to the extent of parenchymal collapse and the rate of deterioration per year in pulmonary function. (Takahashi et al., see above).

Surfactant protein B has so far not been investigated as biomarker for diagnosis or diseases progression of IPF. Reason for this might be that surfactant protein B is, like surfactant protein D, are hydrophobic surfactant protein while surfactant protein A and D are hydrophilic. It is assumed that the hydrophilic water soluble surfactant proteins A and D (SP-A and SP-D) are important for host defense, but have less impact than the hydrophobic surfactant proteins on the biophysical properties of the alveoli and which are also critical for optimizing surface tension reduction. This may also explain the observation of Takahashi that SP-A and SP-D did not correlate with fibrosis which is one of the main features of ILDs and IPF.

The technical problem underlying the present invention can be seen as the provision of means and methods for reliably and efficiently monitoring interstitial lung disease (ILD). The technical problem is solved by the embodiments characterized in the claims and herein below.

Accordingly, the present invention relates to a method for monitoring interstitial lung disease (ILD) in a patient having interstitial lung disease, said method comprising
(a) measuring the level of the biomarker C-terminal proSP-B or proSP-B in a first sample from the patient,
(b) measuring the level of the biomarker C-terminal proSP-B or proSP-B in a second sample from the patient that has been obtained after the first sample, and
(c) comparing the level of the biomarker in the second sample to the level of the biomarker in the first sample.

Preferably, said interstitial lung disease is monitored by carrying the further step of d) monitoring whether said interstitial lung disease has deteriorated or improved based on the results of the comparison carried out in c).

In an embodiment, the level of the biomarker as referred to herein is measured by contacting the sample with an agent that specifically binds to the respective marker, thereby forming a complex between the agent and said marker, detecting the amount of complex formed, and thereby measuring the level of said biomarker.

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the measurement in steps (a) or (b) or a computer-implemented comparison and/or assessment based on said comparison in step (c).

The "patient" as referred to herein is, preferably, a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the patient is a human. The terms "subject" and "patient" are used interchangeably herein.

In accordance with the present invention, the patient shall suffer from interstitial lung disease (abbreviated "ILD"). The term "interstitial lung disease" is well known by the skilled person. Interstitial lung disease (ILD) refers to a group of lung diseases affecting the interstitium, i.e. the tissue and space around the air sacs of the lungs. It may concern alveolar epithelium, pulmonary capillary endothelium, basement membrane, perivascular and peri-lymphatic tissues. Interstitial lung diseases (ILDs) are characterized by progressive shortness of breath which starts at an early disease stage during physical exercise and deteriorates towards shortness at rest and nonproductive cough when the disease is more advanced. The shortness of breath results from damage to the lining of the alveoli leading to inflammation and fibrosis of the interstitium.

The ILD may be classified according to the cause. The cause for the alveoli damage varies and more than 300 different entities if interstitial lung diseases are known.

The ILD may be one of the following diseases: Acute Interstitial Pneumonia (AIP or Hamman-Rich Syndrome), Asbestosis, Bronchiolitis, Connective Tissue-related ILD, Cryptogenic Organizing Pneumonia (COP), Desquamative Interstitial Pneumonia (DIP), Diffuse Alveolar Hemorrhage (DAH), Drug-related ILD, Eosinophilic Pneumonia, Familial Pulmonary Fibrosis, Hypersensitivity Pneumonitis, Idiopathic Pulmonary Fibrosis (IPF), Langerhan's Cell Histiocytosis (aka Eosinophilic granuloma or Histiocytosis X), Lymphangioleiomyomatosis (LAM), Lymphocytic Interstitial Pneumonia (LIP), Non-specific Interstitial Pneumonia (NSIP), Pulmonary Alveolar Proteinosis (PAP), Radiation Fibrosis, Respiratory Bronchiolitis-related ILD (RB-ILD), Sarcoidosis, Silicosis, or Wegener's granulomatosis.

In an embodiment of the present invention, the term "interstitial lung disease" refers to idiopathic interstitial pneumonia. Idiopathic interstitial pneumonias are IDLs of unknown etiology. Preferred idiopathic interstitial pneumonias are idiopathic pulmonary fibrosis (IPF) and sarcoidosis.

In an embodiment, the ILP is idiopathic pulmonary fibrosis (IPF). IPF may be defined as a specific form of chronic progressively fibrosing IIP of unknown cause that occurs primarily in relatively elderly adults. It is the most pernicious and frequent of the ILDs. IPF is associated with a characteristic radiological and/ or histopathological pattern of usual interstitial pneumonia (UIP). How to diagnose IPF is, e.g. described in Raghu G et al. Am J Respir Crit Care Med (2011) 183, 788-824 which is herewith incorporated by reference with respect to its entire disclosure content. Imaging criteria for the diagnosis of a UIP pattern on high resolution computed tomography (HRCT) include the presence of bilateral, predominantly subpleural, basal reticular abnormalities and the absence of additional features considered incompatible with a diagnosis of IPF. The definite diagnosis requires the presence of honeycombing which present as clustered cystic air space, usually of comparable diameters (3-10 mm, occasionally larger), with well-defined thickened walls and mainly subpleural. If diagnosis cannot be achieved by HRCT, surgical lung biopsy might be applied.

In the context of the present invention, it is contemplated that the patient to be tested has a mild form or severe form of ILD, in particular of IPF, in particular at the time point at which the first and/or at the time point at which the second sample is obtained.

Preferably, it is envisaged that the patients has severe form of ILD, in particular of IPF. It is envisaged that the patient has a severe form of ILD at the time point at which the first sample is obtained and/or at the time point at which the second sample is obtained. Thus, the patient may have a severe form of ILD at one of the two time points (either the first or the second time point), or in particular, at both time points. In this case, the marker to be tested is, preferably, C-terminal proSP-B since it has been shown in the context of the present invention that C-terminal proSP-B allows for an even more reliable assessment in patients with a severe form of ILD than proSP-B.

Whether a patient suffers from a mild or severe form of ILD can be assessed by the skilled person without further ado e.g. by lung function testing, see e.g. Miller MR, Crapo R, Hankinson J, et al. ATS/ERS Task Force. General considerations for lung function testing. Eur Respir J. 2005; 26:153-61 Eur Respir J. 2005; 26:153-61.

A patient who has severe form of ILD, in particular of IPF, preferably, has a FEV1 of lower than 50%, more preferably, of lower than 45%, and most preferably, of lower than 40%. Alternatively or additionally, a patient who has severe form of ILD, in particular of IPF, preferably, has a TLCO (Transfer factor of the lung for carbon monoxide) of lower than 50% or lower than 40%. In an embodiment, the patient thus may have a FEV1 of lower than 50% and a TLCO of lower than 40%. Also preferably, a patient who has a severe form of ILD, in particular of IPF, has a serum level of C-terminal pro-SP-B of more than 2000 ng/ml at the time point at which the first sample and/or at the time point at which the second sample is obtained.

A patient who has a mild form of ILD, in particular of IPF, preferably, has a FEV1 of larger than 65%, more preferably, of larger than 70%, and most preferably, of larger than 75%. Alternatively or additionally, a patient who has mild form of ILD, in particular of IPF, preferably, has a TLCO (Transfer factor of the lung for carbon monoxide) of larger than 60% or lower than 70%. In an embodiment, the patient thus may have a FEV1 of larger than 70% and a TLCO of larger than 70%.

FEV1 [%] is the forced expiratory volume in 1 second, i.e. the relative amount of air which can be forcibly exhaled from the lungs in the first second of a forced exhalation. It can be determined by well known methods, preferably, by spirometry. The TLCO is the Transfer factor of the lung for carbon monoxide.

In an embodiment, the FEV1 and/or the TLCO is/are determined as disclosed by R. Pellegrino et al., Interpretative strategies for lung function tests Eur Respir Rev, 2013; 22: 128, 158-162).

The term "monitoring" as used herein, preferably, refers to assessing the course of ILD, and in particular of IPF. Preferably, by assessing the course of ILD, it can be determined whether the condition of a patient with respect to the disease improves or deteriorates.

As will be understood by those skilled in the art, such an assessment is usually not intended to be correct for 100% of the patients to be monitored. The term, however, requires that the assessment is correct for a statistically significant portion of the patients (e.g. a cohort in a cohort study). Whether a portion is statistically significant can be determined without further ado by the person skilled in the art using various well known statistic evaluation tools, e.g., determination of confidence intervals, p-value determination, Students t-test, Mann- Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1 , 0.05, 0.01 , 0.005, or 0.0001.

Whether the condition of a patient, i.e. the condition of the patient regarding ILD improves can be confirmed by methods well known in art (see e.g. Examples section).

The term "sample" refers to a sample of a body fluid, to a sample of separated cells or to a sample from a tissue or an organ. Samples of body fluids can be obtained by well known techniques and include, preferably, samples of blood, plasma, serum, or urine. It is also envisaged that the sample is bronchoalveolar lavage (BAL) sample. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Preferred samples are blood, plasma, serum samples. Also preferred is a bronchoalveolar lavage (BAL) sample.

The "first sample" can be obtained from the patient at any time after the onset of ILD. As set forth above, the patient may suffer from a mild or severe form of ILD.

The "second sample" shall be obtained after the first sample. The sample is, preferably, understood as a sample which is obtained in order to reflect a change of the level of the respective marker as compared to the level of the respective marker in the first sample. Preferably, the second sample is not obtained too early after the first sample (in order to observe a sufficiently significant change to allow for monitoring). Preferably, the second sample is, thus, obtained at least one month, more preferably, at least three months, or most preferably, at least one year after the first sample.

It is also envisaged that the second sample is obtained at least one month, but not more than three months, more preferably, at least 3 months but not more than 6 months, or most preferably, at least 9 months but not more than 24 months after the first sample.

In particular, the "second sample" is, thus, obtained within one to three months, more preferably within 3 to 6 months, or within 9 to 24 months after the first sample.

It is also contemplated to obtain said second sample 1 month, 3 months, or 12 months after said first sample.

Short intervals between the samples (such as at least one month, but not more than three months) advantageously allow for the assessment of acute deterioration of ILD.

The biomarkers C-terminal proSP-B and proSP-B are well known in the art and e.g. are disclosed in WO 2012/130731 which herewith is incorporated by reference with respect to the entire disclosure content, in particular with respect to the biomarkers C-terminal proSP-B and proSP-B as well as the sequences thereof.

Surfactant Protein B (SP-B) is a 79-amino acid peptide, produced by the proteolytic cleavage of SP-B proprotein (proSP-B; for a preferred sequence of the biomarker proSP-B, see e.g. SEQ ID NO: 1 of WO 2012/130731) that is processed to a smaller, lipid-associated peptide in the distal secretory pathway within the type II cell. Processing of proSP-B occurs in the multivesicular body (MVB) and lamellar body (LB) compartments.

SP-B facilitates the stability and rapid spreading of surfactant phospholipids during respiratory cycles. It maintains the molecular continuity of the monolayer of the lipid and peptide at the air-water interface during breathing and facilitates the incorporation of lipid from the lung aqueous subphase into the lipid monolayer at the alveolar air-water interface. Processing of the SP-B preproprotein to its mature peptide occurs during transit through the secretory pathway from the endoplasmic reticulum to the Golgi network and the multivesicular bodies in the type II epithelial cell.

Entry of the SP-B preproprotein into the secretory pathway is mediated by the N-terminal signal peptide which is cleaved upon translation of the proprotein into the endoplasmic reticulum. Transit of SP-B out of the endoplasmic reticulum is dependent on the N-terminal propeptide, which likely facilitates folding and/or sequestration of the hydrophobic mature peptide. The C-terminal propeptide (amino acids 280 to 381 of proSP-B = SEQ ID NO: 2 of WO 2012/130731) however, seems not to be required for sorting of SP-B to secretory granules.

C-terminal proSP-B in the sense of the present invention, preferably, relates to proSP-B and all cleavage products or fragments thereof comprising the C-terminal sequence of proSP-B. Thus, the measurement of the level of C-terminal proSP-B may encompass the measurement of any polypeptide which comprises said C-terminal sequence. In an embodiment, the C-terminal sequence encompasses about 100 amino acids, in particular 101 amino acids at the C-terminus of proSP-B. In particular, the C-terminal sequence may span from amino acids 280 to 381 of proSP-B (see SEQ ID NO: 2 in WO 2012/130731 which shows these 101 amino acids). In another embodiment, the C-terminal sequence may span from amino acids 285 to 334 of proSP-B (for this region see SEQ ID NO: 3 in WO 2012/130731).

It is preferred that the C-terminal sequence is the sequence as defined in SEQ ID NO: 3 of WO 2012/130731. Accordingly, the measurement of C-terminal proSP-B thus relates in particular to the measurement of proSP-B and those fragments or cleavage products thereof comprising SEQ ID NO:3 as disclosed WO 2012/130731. In the present invention C-terminal proSP-B thus may encompass proSP-B and those proSP-B fragments comprising the C-terminal sequence of proSP-B as defined in SEQ ID NO:3 of WO 2012/130731.

C-terminal proSP-B may include but is not limited to the following: proSP-B (i.e. the proSP-B of SEQ ID NO:1 of WO 2012/130731, comprising the N-terminal propeptide sequence the sequence stretch representing the mature SP-B and the C-terminal pro SP-B); the mid-molecular plus C-terminal fragment (i.e. the amino acids from position 201 to 381 of SEQ ID NO:1 of WO 2012/130731) and the C-terminal proSP-B fragment (i.e. the amino acids from position 280 to 381 of SEQ ID NO:1 of WO 2012/130731). As obvious to the artisan, proSP-B and fragments comprising the C-terminal proSP-B sequence of SEQ ID NO: 3 of WO 2012/130731 may be present as monomers and/or dimers, respectively.

For determination of C-terminal proSP-B the sample obtained from an individual is incubated in vitro with the specific binding agent for C-terminal proSP-B under conditions appropriate for formation of a binding agent C-terminal proSP-B complex. Such conditions need not be specified, since the skilled artisan without any inventive effort can easily identify such appropriate incubation conditions. As the skilled artisan will appreciate there are numerous methods to determine the level of the specific binding agent C-terminal proSP-B complex all described in detail in relevant textbooks (cf., e.g., Tijssen, P., supra, or Dia-mandis, E.P., and Christopoulos, T.K. (eds.), Immunoassay, Academic Press, Boston (1996)).

Assays for the determination of the level of proSP-B and C-terminal proSP-B are disclosed in Examples 1 and 2, respectively, of WO 2012/130731 which are both incorporated by reference as well. In an embodiment, the biomarkers are measured as described in these examples.

The biomarkers as referred to herein, C-terminal pro-SP-B or pro-SP-B can be detected using methods generally known in the art. Methods of detection generally encompass methods to quantify the level of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence-based immunoassays, which are commercially available. Further suitable methods to detect biomarker include measuring a physical or chemical property specific for the peptide or polypeptide such as its precise molecular mass or NMR spectrum. Said methods comprise, e.g., biosensors, optical devices coupled to immunoassays, biochips, analytical devices such as mass- spectrometers, NMR- analyzers, or chromatography devices. Further, methods include microplate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used immunoassays.

Methods for measuring electrochemiluminescent phenomena are well-known. Such methods make use of the ability of special metal complexes to achieve, by means of oxidation, an excited state from which they decay to ground state, emitting electrochemiluminescence. For review see Richter, M.M., Chem. Rev. 104 (2004) 3003-3036.

Biomarkers can also be detected by generally known methods including magnetic resonance spectroscopy (NMR spectroscopy), Gas chromatography-mass spectrometry (GC-MS), Liquid chromatography-mass spectrometry (LC-MS), High and ultra-HPLC HPLC such as reverse phase HPLC, for example, ion-pairing HPLC with dual UV-wavelength detection, capillary electrophoresis with laser-induced fluorescence detection, anion exchange chromatography and fluorescent detection, thin layer chromatography.

Preferably, measuring the level of a C-terminal proSP-B or proSP-B comprises the steps of (a) contacting a cell capable of eliciting a cellular response the intensity of which is indicative of the level of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the level of the peptide or polypeptide.

Also preferably, measuring the level of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Measuring the level of a peptide or polypeptide may, preferably, comprises the steps of (a) contacting the peptide with a specific binding agent, (b) (optionally) removing non-bound binding agent, (c) measuring the level of bound binding agent, i.e. the complex of the binding agent formed in step(a). According to a preferred embodiment, said steps of contacting, removing and measuring may be performed by an analyzer unit of the system disclosed herein. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The bound binding agent, i.e. the binding agent or the binding agent/peptide complex, will generate an intensity signal. Binding according to the present invention includes both covalent and non-covalent binding. A binding agent according to the present invention can be any compound, e.g., a peptide, polypeptide, nucleic acid, or small molecule, binding to the peptide or polypeptide described herein. Preferred binding agents include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such binding agents are well-known in the art. For example, identification and production of suitable antibodies or aptamers is also offered by commercial suppliers. The person skilled in the art is familiar with methods to develop derivatives of such binding agents with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the binding agent or agent binds specifically to the pep-tide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the binding agent can be measured by any method known in the art. Preferably, said method is semi-quantitative or quantitative. Further suitable techniques for the determination of a polypeptide or peptide are de-scribed in the following.

Binding of a binding agent may be measured directly, e.g. by NMR or surface plasmon resonance. Measurement of the binding of a binding agent, according to preferred embodiments, is performed by an analyzer unit of a system disclosed herein. Thereafter, a level of the measured binding may be calculated by a computing device of a system disclosed herein. If the binding agent also serves as a substrate of an enzymatic activity of the pep-tide or polypeptide of interest, an enzymatic reaction product may be measured (e.g. the level of a protease can be measured by measuring the level of cleaved substrate, e.g. on a Western Blot). Alternatively, the binding agent may exhibit enzymatic properties itself and the "binding agent/peptide or polypeptide" complex or the binding agent which was bound by the peptide or polypeptide, respectively, may be contacted with a suitable substrate allowing detection by the generation of an intensity signal. For measurement of enzymatic reaction products, preferably the level of substrate is saturating. The substrate may also be labeled with a detectable label prior to the reaction. Preferably, the sample is contacted with the substrate for an adequate period of time. An adequate period of time refers to the time necessary for a detectable, preferably measurable, level of product to be produced. Instead of measuring the level of product, the time necessary for appearance of a given (e.g. detectable) level of product can be measured. Third, the binding agent may be coupled covalently or non-covalently to a label allowing detection and measurement of the binding agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of tertiary binding agent binding to the secondary binding agent. The use of secondary, tertiary or even higher order binding agents is often used to increase the signal. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus hae-magglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, avail-able as ready-made stock solution from Roche Diagnostics), CDP-Star™ (Amersham Bio-sciences), ECF™ (Amersham Biosciences). A suitable enzyme-substrate combination may result in a colored reaction product, fluorescence or chemoluminescence, which can be measured according to methods known in the art (e.g. using a light-sensitive film or a suit-able camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The level of a peptide or polypeptide may be, also preferably, determined as follows: (a) contacting a solid support comprising a binding agent for the peptide or polypeptide as specified above with a sample comprising the peptide or polypeptide and (b) measuring the level peptide or polypeptide which is bound to the support. The binding agent, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The binding agent or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said binding agent are well known and include, but are not limited to ionic, hydrophobic, covalent interactions and the like. It is also contemplated to use "suspension arrays" as arrays according to the present invention (No-lan 2002, Trends Biotechnol. 20(1):9-12). In such suspension arrays, the carrier, e.g. a microbead or microsphere, is present in suspension. The array consists of different microbeads or microspheres, possibly labeled, carrying different binding agents. Methods of producing such arrays, for example based on solid-phase chemistry and photo-labile protective groups, are generally known (US 5,744,305).

In an embodiment of the method of the present invention, the levels of the biomarkers as referred to herein are measured by using the assays described in the Examples section.

In another embodiment of the method of the present invention, the measurement in step a) may be carried out by an analyzer unit, in particular by an analyzer unit as defined elsewhere herein.

The term "binding agent" refers to a molecule that comprises a binding moiety which specifically binds the corresponding to the respective biomarker.

The term "specific binding" or "specifically bind" refers to a binding reaction wherein binding pair molecules exhibit a binding to each other under conditions where they do not significantly bind to other molecules. The term "specific binding" or "specifically binds", when referring to a protein or peptide as biomarker, refers to a binding reaction wherein a binding agent binds to the corresponding biomarker with an affinity of at least 10⁻⁷ M. The term "specific binding" or "specifically binds" preferably refers to an affinity of at least 10⁻⁸ M or even more preferred of at least 10⁻⁹ M for its target molecule. The term "specific" or "specifically" is used to indicate that other molecules present in the sample do not significantly bind to the binding agent specific for the target molecule. Preferably, the level of binding to a molecule other than the target molecule results in a binding affinity which is only 10% or less, more preferably only 5% or less of the affinity to the target molecule.

Examples of "binding agents" are a nucleic acid probe, nucleic acid primer, DNA molecule, RNA molecule, aptamer, antibody, antibody fragment, peptide, peptide nucleic acid (PNA) or chemical compound. A preferred binding agent is an antibody which specifically binds to the biomarker to be measured. The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity. Preferably, the antibody is a polyclonal antibody. More preferably, the antibody is a monoclonal antibody. Moreover, preferred antibodies for the determination of C-terminal proSP-B are disclosed in WO 2012/130731

Another binding agent that can be applied, in an aspect, may be an aptamere which specifically binds to the at least one marker in the sample. The term "specific binding" or "specifically binds", when referring to a nucleic acid aptamer as a binding agent, refers to a binding reaction wherein a nucleic acid aptamer binds to the corresponding target molecule with an affinity in the low nM to pM range.

In yet an aspect the, sample is removed from the complex formed between the binding agent and the at least one marker prior to the measurement of the level of formed complex. Accordingly, in an aspect, the binding agent may be immobilized on a solid support. In yet an aspect, the sample can be removed from the formed complex on the solid support by applying a washing solution. The formed complex shall be proportional to the level of the at least one marker present in the sample. It will be understood that the specificity and/or sensitivity of the binding agent to be applied defines the degree of proportion of at least one marker comprised in the sample which is capable of being specifically bound. Further details on how the determination can be carried out are also found elsewhere herein. The level of formed complex shall be transformed into a level of at least one marker reflecting the level indeed present in the sample. Such a level, in an aspect, may be essentially the amount present in the sample or may be, in another aspect, an amount which is a certain proportion thereof due to the relationship between the formed complex and the amount present in the original sample.

The term "level" as used herein encompasses the absolute amount of a biomarker as referred to herein, the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response amounts determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the level of a biomarker as referred to herein in a first sample with a level of said marker in a second sample. The terms "first sample" and "second sample" are specified elsewhere in this description. It is to be understood that comparing as used herein refers to a comparison of corresponding parameters or values. It is to be understood that a level of a biomarker is a first sample is compared to a level of the respective marker, thus the same marker, in a second sample. The comparison referred to in the context of the method of the present invention may be carried out manually or computer assisted. For a computer assisted comparison, the value of the determined level in the second sample may be com- pared to a value of the determined level in a first sample which is stored in a database by a computer program. The computer program may further evaluate the result of the comparison, i.e. automatically provide the desired assessment in a suitable output format. Based on the comparison of the level of a biomarker as referred to herein in a first sample to the level in a second sample it is possible to monitor ILD. As set forth above, the monitoring is, preferably, based on the comparison of the level of the biomarker in a first sample to the level of the said biomarker in a second sample obtained after said first sample.

Preferably, an increase, and more preferably a statistically significant increase of the level of the biomarker in said second sample compared to the level in said first sample indicates deterioration of ILD, and, thus, that the ILD has deteriorated during the period between obtaining the first and the second sample. Thus, there was a progression of ILD.

Preferably, a decrease of the level of the biomarker in said second sample as compared to said first sample indicates amelioration of said ILD, and, thus, that the inflammatory pulmonary disease has improved during the period between obtaining the first and the second sample. Thus, there was regression of ILD.

Particularly, a significant increase (or decrease) is an increase (or decrease) of a size which is considered to be significant for, particularly statistically significant. The terms "significant" and "statistically significant" are known to the person skilled in the art. Whether a change or an increase (or decrease) is statistically significant can be determined without further ado by the person skilled in the art using various well known statistic evaluation tools including those referred to herein.

Moreover, an increase of the level in the second sample as compared to the level in the first sample which is considered to be significant is an increase of the level in the second sample as compared to the first sample in a patient or a group of patients in which the ILD has deteriorated. Moreover, a decrease of the level in the second sample as compared to the level in the first sample which is considered to be significant is a decrease of the level in the second sample as compared to the first sample in a patient or a group of patients in which the ILD has improved.

Moreover, an unchanged level, preferably an essentially unchanged level, in said second sample as compared to said first sample, preferably, indicates no change in status of said ILD. Thus, the condition of the patient was stable.

Preferred significant increases of the levels of the biomarker which have been found in the course of the invention which indicate that ILD has deteriorated are given below. According to the invention, an increase of the biomarker level in the second sample compared to the level in the first sample, preferably, of at least 10% more preferably of at least 30 % and most preferably, of at least 50 % considered to be significant and, thus, to be indicative for a deterioration of ILD.

Moreover, an increase of the level of the biomarker in the second sample compared to the level in the first sample, preferably, of at least 50 ng/ml, more preferably of at least 100 ng/ml and even, more preferably, of at least 200 ng/ml, and most preferably of at least 500 ng/ml is considered to be significant and, thus, to be indicative for a deterioration of ILD, if the biomarker is C-terminal proSP-B. If the biomarker is proSP-B, an increase of the level of the biomarker in the second sample compared to the level in the first sample, preferably, of at least 30 ng/ml, more preferably of at least 70 ng/ml and even, more preferably, of at least 100 ng/ml, and most preferably of at least 150 ng/ml is considered to be significant and, thus, to be indicative for a deterioration of ILD.

Preferably, a decrease of the level of the in the second sample compared to the level in the first sample, preferably, of at least 10%, more preferably of at least 20 % and most preferably of at least 30% is considered to be significant and, thus, to be indicative for an amelioration/improvement of ILD.

Moreover, a decrease of the level of the biomarker in the second sample compared to the level in the first sample, preferably, of at least 50 ng/ml, more preferably of at least 100 ng/ml and even, more preferably, of at least 200 ng/ml, and most preferably of at least 300 ng/ml or 500 ng/ml is considered to be significant and, thus, to be indicative for an amelioration/improvement of ILD, if the biomarker is C-terminal proSP-B. If the biomarker is proSP-B a decrease of the level of biomarker in the second sample compared to the level in the first sample, preferably, of at least 30 ng/ml, more preferably of at least 70 ng/ml and even, more preferably, of at least 100 ng/ml, and most preferably of at least 150 ng/ml is considered to be significant and, thus, to be indicative for an amelioration/improvement of ILD.

If the method shows a deterioration of ILD, then the subject may be treated accordingly. Preferred treatments against ILD are disclosed herein below (see the next method as well as Example 3).Accordingly, the present invention also envisages a method of treatment.

Advantageously, it has been found in the studies underlying the present invention that i) the determination of the level of C-terminal proSP-B or proSP-B in a first sample and in a second sample of a patient suffering from ILD and ii) the comparison of the level of the marker in said first sample to the level in said second sample allows for reliably monitoring ILD, in particular IPF. It was found that an increase of the biomarker in a second sample as compared to a first (earlier obtained) sample indicates deterioration of ILD, whereas a decrease of C-terminal proSP-B or proSP-B in a second sample as compared to a first (earlier obtained) sample indicates amelioration of ILD (see accompanying Examples, below). Thanks to the present invention, it is possible to more reliably monitor ILD. Moreover, the time consuming, expensive and cumbersome diagnostic measures can be avoided when applying the method of the invention as an aid for diagnosis.

Also advantageously, the present invention allows for an identification of slow and large progressors of ILD, in particular of IPF. In this case, the second sample is preferably obtained at least six months or, in particular, at least 12 months after the first sample. A moderate increase of the level of the biomarker in the second sample as compared to the level of the biomarker in the first sample preferably is indicative for a patient who is a slow progressor of ILD, whereas a strong increase preferably is indicative for a patient who is a fast progressor of ILD. The person skilled in the art understands the terms "moderate" and "strong" in this context and can determine a moderate and a strong increase without further ado. A moderate increase is, preferably, an annualized increase within a range from about 5% to 20%, in particular within a range from 5 to 15%. A strong increase is preferably, an annualized increased of more than 20%, more preferably, of more than 25%, and, most preferably, of more than 30%.

If the marker is C-terminal proSP-B, an annualized increase within a range from about 50 ng/ml to 200 ng/ml, in particular from about 50 ng/ml to 150 ng/ml, or 50 ng/ml to 100 ng/ml is considered to be moderate, whereas an annualized increase of larger than 300 ng/ml, in particular of larger than 450 ng/ml or larger than 600 ng/ml is considered to be strong.

The definitions given herein above apply *mutatis mutandis* to the following.

### Method for assessing whether a patient responds to a therapy against interstitial lung disease.

Advantageously, it has been shown in the studies carried out in the context of the present invention that the determination of C-terminal proSP-B or proSP-B allows for the identification of patients who respond to a therapy against interstitial lung disease, or not.

The present invention, thus, relates to a method of assessing whether a patient having interstitial lung disease responds to a therapy against interstitial lung disease (ILD), the method comprising
(a) measuring the level of C-terminal proSP-B or proSP-B in a first sample from the patient,
(b) measuring the level of C-terminal proSP-B or proSP-B in a second sample from the patient that has been obtained after the first sample, and
(c) comparing the level of C-terminal proSP-B or proSP-B in the second sample to the level in the first sample.

Preferably, it is assessed whether the patient responds to said therapy by carrying the further step of d) assessing whether said patients responds to said therapy based on the results of the comparison carried out in step (c).

Alternatively, the method may comprise step (d) of providing an assessment whether the patient responds to said therapy based on the comparison carried out in step (c).

Also envisaged is a method for identifying a patient who responds to a therapy against interstitial lung disease (ILD), the method comprising
(a) measuring the level of C-terminal proSP-B or proSP-B in a first sample from the patient,
(b) measuring the level of C-terminal proSP-B or proSP-B in a second sample from the patient that has been obtained after the first sample, and
(c) comparing the level of C-terminal proSP-B or proSP-B in the second sample to the level in the first sample.

Preferably, a decrease of the level of the biomarker in said second sample as compared to said first sample, or an essentially unchanged level in said second sample as compared to said first sample indicates that the patients responds to said therapy. Also preferably, an increase of the level in said second sample as compared to said first sample indicates that the patient does not respond to said therapy.

The information or data used or generated for the identification or assessment may be in any form, written, oral or electronic. In some embodiments, using the information or data generated includes communicating, presenting, reporting, storing, sending, transferring, supplying, transmitting, dispensing, or combinations thereof. In some embodiments, communicating, presenting, reporting, storing, sending, transferring, supplying, transmitting, dispensing, or combinations thereof are performed by a computing device, analyzer unit or combination thereof. In some further embodiments, communicating, presenting, reporting, storing, sending, transferring, supplying, transmitting, dispensing, or combinations thereof are performed by a laboratory or medical professional. In some embodiments, the information or data includes an indication that the patient responds to the therapy, or not.

As will be understood by those skilled in the art, the assessment whether a patient is likely to respond to the therapy against ILD is usually not intended to be correct for 100% of the patients to be assessed. The term, however, requires that the assessment is correct for a statistically significant portion of the patients (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the patients of a population can be properly identified by the method of the present invention.

The term "interstitial lung disease" has been defined above in connection with the method for monitoring ILD. The same definition applies. In an embodiment, the interstitial lung disease is idiopathic pulmonary fibrosis (IPF)

The term "patient" has been also defined above. The same definition applies. As set forth above, the patient may have mild form of ILD, or a severe form of ILD. In an embodiment the biomarker is C-terminal proSP-B and the patient has a severe form of ILD, in particular, IPF (either at one time point at which the sample is obtained or at both time points, see above).

The phrase "identifying a patient" as used herein, preferably, refers to using the information or data generated relating to the level of the at least one marker as referred to herein in a sample of a patient to identify or selecting the patient as to respond to said therapy against ILD, or not. In particular, a patient is considered to respond to said therapy, if the condition of said patient as regards to ILD improved in the interval between the first and second sample, or remained essentially unchanged, i.e. stable, in this interval. In this case, the therapy can be continued. A patient is considered not to respond to said therapy, if the condition of said patient as regards to ILD deteriorated in this interval. In this case, the therapy can be stopped, changed and/or adapted. Thereby, unnecessary health care costs can be avoided. Further, adverse side effects that may result from the therapy can be avoided.

The therapy against interstitial lung disease (ILD) may be any therapy that can be used for treating ILD, in particular, IPF. Such therapies are well known in the art. Since ILD is not a single disease, but encompasses many different pathological processes, treatment is different for each disease. A preferred therapy against ILD, in particular, IPF is treatment with a corticosteroid, in particular prednisolone or treatment with immunosuppressants such as cortisone. Another therapy is treatment with Pirfenidone. It is also envisaged that the therapy is treatment with antifibrotic or antioxidant drugs. Further preferred therapies are described in the Examples section or by Raghu G et al. Am J Respir Crit Care Med (2011) 183, 788-824 which is herewith incorporated by reference with respect to its entire disclosure content.

In an embodiment, the therapy is lung transplantation.

The term "first sample" and "second sample" were defined above. The definition applies. The patient to be tested may be treated against ILD at the time point at which the first sample has been obtained, or not. If the patient is not treated, the therapy against ILD is preferably initiated no later than one week or three days after the first sample has been obtained.

Preferred decreases (or increases) of the level of the biomarker to be measured in accordance with the aforementioned method which are indicative for a patient who does respond against ILD (who does not respond to a therapy against ILD), are the decreases (increases) given herein above which are indicative for an improvement of ILD (for a deterioration of ILD), please see the method for monitoring ILD.

### Method for identifying a patient as likely to show improvement of interstitial lung disease

In addition, it was shown in the context of the studies underlying the present invention, that a patient who has ILD is more likely to show improvement of ILD if the level of C-terminal proSP-B and/or proSP-B is decreased, wherein the patient is less likely to show improvement of ILD if the reference level of C-terminal proSP-B and/or proSP-B is increased.

Accordingly, the present invention also relates method for identifying a patient suffering from interstitial lung disease (ILD) as likely to show improvement of interstitial lung disease, comprising the step of
(a) measuring the level of the biomarker C-terminal proSP-B or of the biomarker proSP-B in a sample from the patient, and
(b) comparing the level measured in (a) to a reference level for said biomarker, wherein a level of the biomarker below the reference level indicates that the patient is more likely to show improvement of ILD, and/or wherein a level of the biomarker above the reference level indicates that the patient is less likely to show improvement of ILD.

The present invention also relates to the use of i) the biomarker C-terminal proSP-B or proSP-B and/or of ii) of a binding agent which specifically binds to the biomarker C-terminal proSP-B or proSP-B in a first and a second sample from a patient having interstitial lung disease (ILD) for monitoring interstitial lung disease (ILD) in said patient or for assessing whether said patient responds to a therapy against interstitial lung disease. Also envisaged is the use of i) the biomarker C-terminal proSP-B or proSP-B and/or of ii) of a binding agent which specifically binds to the biomarker C-terminal proSP-B or proSP-B in a sample from a patient having interstitial lung disease (ILD) for identifying a patient as likely to show improvement of interstitial lung disease.

The present invention also relates to the use of i) the biomarker C-terminal proSP-B or proSP-B and/or of ii) of a binding agent which specifically binds to the biomarker C-terminal proSP-B or proSP-B for the manufacture of a pharmaceutical or diagnostic composition for a) monitoring interstitial lung disease (ILD) in a patient or for b) assessing whether a patient responds to a therapy against interstitial lung disease in a first and a second sample from a patient having interstitial lung disease (ILD). Also envisaged is the use of i) the biomarker C-terminal proSP-B or proSP-B and/or of ii) of a binding agent which specifically binds to the biomarker C-terminal proSP-B or proSP-B for the manufacture of a pharmaceutical or diagnostic composition for identifying a patient who has ILD as likely to show improvement of interstitial lung disease.

According to a preferred embodiment of the present invention, a device adapted for carrying out a method of the invention is provided comprising
a) an analyzer unit comprising a binding agent which specifically binds to the biomarker C-terminal proSP-B or to proSP-B, said unit being adapted for measuring the level of the biomarker in a first and second sample of a patient having ILD, and
b) an analyzer unit for comparing the determined levels in the first and second sample, whereby interstitial lung disease is monitored or whereby it is assessed whether said patient responds to a therapy against interstitial lung disease, and a computer-implemented algorithm for carrying out the comparison.

Preferred increases/decreases which are indicative for the monitoring/assessment and algorithms are disclosed herein above.

A preferred embodiment of the instant disclosure includes a system for identifying a patient as likely to respond to a therapy comprising a statin. Examples of systems include clinical chemistry analyzers, coagulation chemistry analyzers, immunochemistry analyzers, urine analyzers, nucleic acid analyzers, used to detect the result of chemical or biological reactions or to monitor the progress of chemical or biological reactions. More specifically, exemplary systems of the instant disclosure may include Roche Elecsys™ Systems and Cobas^{®} e Immunoassay Analyzers, Abbott Architect™ and Axsym™ Analyzers, Siemens Centaur™ and Immulite™ Analyzers, and Beckman Coulter UniCel™ and Acess™ Analyzers, or the like.

Embodiments of the system may include one or more analyzer units utilized for practicing the subject disclosure. The analyzer units of the system disclosed herein are in operable communication with the computing device disclosed herein through any of a wired connection, Bluetooth, LANS, or wireless signal, as are known. Additionally, according to the instant disclosure, an analyzer unit may comprise a stand-alone apparatus, or module within a larger instrument, which performs one or both of the detection, e.g. qualitative and/or quantitative evaluation of samples for diagnostic purpose. For example, an analyzer unit may perform or assist with the pipetting, dosing, mixing of samples and/or reagents. An analyzer unit may comprise a reagent holding unit for holding reagents to perform the assays. Reagents may be arranged for example in the form of containers or cassettes containing individual reagents or group of reagents, placed in appropriate receptacles or positions within a storage compartment or conveyor. Detection reagents may also be in immobilized form on a solid support which are contacted with the sample. Further, an analyzer unit may include a process and/or detection component which is optimizable for specific analysis.

According to some embodiments, an analyzer unit may be configured for optical detection of an analyte, for example a marker, with a sample. An exemplary analyzer unit configured for optical detection comprises a device configured for converting electro-magnetic energy into an electrical signal, which includes both single and multi-element or array optical detectors. According to the present disclosure, an optical detector is capable of monitoring an optical electro-magnetic signal and providing an electrical outlet signal or response signal relative to a baseline signal indicative of the presence and/or concentration of an analyte in a sample being located in an optical path. Such devices may also include, for example, photodiodes, including avalanche photodiodes, phototransistors, photoconductive detectors, linear sensor arrays, CCD detectors, CMOS detectors, including CMOS array detectors, photomultipliers, and photomultiplier arrays. According to certain embodiments, an optical detector, such as a photodiode or photomultiplier, may contain additional signal conditioning or processing electronics. For example, an optical detector may include at least one pre-amplifier, electronic filter, or integrated circuit. Suitable pre-preamplifiers include, for example, integrating, transimpedance, and current gain (current mirror) preamplifiers.

Additionally, one or more analyzer unit according to the instant disclosure may comprise a light source for emitting light. For example, a light source of an analyzer unit may consist of at least one light emitting element (such as a light emitting diode, an electric powered radiation source such as an incandescent lamp, an electroluminescent lamp, a gas discharge lamp, a high-intensity discharge lamp, a laser) for measuring analyte concentrations with a sample being tested or for enabling an energy transfer (for example, through florescent resonance energy transfer or catalyzing an enzyme).

Further, an analyzer unit of the system may include one or more incubation units (for example, for maintaining a sample or a reagent at a specified temperature or temperature range). In some embodiments, an analyzer unit may include a thermocycler, include a real-time thermocycler, for subjecting a sample to repeated temperature cycles and monitoring a change in the level of an amplification product with the sample.

Additionally, an analyzer unit of the system disclosed herein may comprise, or be operationally connected to, a reaction vessel or cuvette feeding unit. Exemplary feeding units include liquid processing units, such as a pipetting unit, to deliver samples and/or reagents to the reaction vessels. The pipetting unit may comprise a reusable washable needle, e.g. a steel needle, or disposable pipette tips. The analyzer unit may further comprise one or more mixing units, for example a shaker to shake a cuvette comprising a liquid, or a mixing paddle to mix liquids in a cuvette, or reagent container.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities. According to some embodiments of the instant disclosure, an algorithm for carrying out a comparison between a determined level of one or more markers disclosed herein, and a suitable reference (in particular, the level in the first sample), is embodied and performed by executing the instructions. The results may be given as output of parametric diagnostic raw data or as absolute or relative levels. According to various embodiments of the system disclosed herein, a "diagnosis" may be provided by the computing device of a system disclosed herein based on said comparison of the calculated "level" to a reference or a threshold, in particular the level in the first sample to the level in the second sample. For example, a computing device of a system may provide an indicator, in the form of a word, symbol, or numerical value which is indicative of a particular diagnosis.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Example 1a: Comparison with surfactant proteins proSP-B/C-terminal proSP-B, SP-B/SP-C/SP-A/SP-D

- Patients: inter alia, pneumonia (n = 5), IPF (n = 5), healthy controls (n = 5)
- Sample types: serum samples; bronchoalveolar lavage fluids
- Measurements: proSP-B/C-terminal proSP-B, SP-B/SP-C/SP-A/SP-D

Similar trends were observed for all surfactant proteins. Interestingly however, proSP-B and in particular, C-terminal proSP-B shows the largest range (not show). Thus, the markers may allow for a more reliable discrimination of patients.

In particular, C-terminal proSP-B showed a broader dynamic range compared to proSP-B or the other surfactant proteins (SP-A, SP-D and SP-C) and may therefore serve as the better biomarker for therapy monitoring compared to the other markers. In a patient cohort of 20 patients diagnosed of IPF, C-terminal proSP-B and proSP-B as well as SP-A, SP-C and SP-D were determined in serum specimens of those patients. While SP-A, SP-C levels were hardly detectable, and SP-D only at very low level, the C-terminal proSP-B levels ranged between 500 at 5000 ng/mL (proSP-B levels to almost 1500 ng/mL).

### Example 1b: Determination of proSP-B and C-terminal proSP-B in pulmonary disease patients

- Patients: patients with various pulmonary diseases (n = 280)
- Sample type: serum
- Measurements: proSP-B and C-terminal proSP-B

Results: The highest levels of the markers were observed in the subgroup of IPF patients (not shown). Since proSP-B and C-terminal proSP-B show the largest range of surfactant markers (see Example 1a) and since the highest levels of theses markers are found in IPF patients, the use of these markers is particularly advantageous in this subgroup. In comparison to pro-SP-B, higher values were found for C-terminal pro-SP-B (not shown).

### Example 2a: Correlation with clinics/clinical presentation, long term monitoring

- Patients: IPF (n=50), 2 time points (baseline and on average 1 year)
- Sample material: serum, bronchoalveolar lavage (BAL)
- Measurements: proSP-B/C-terminal proSP-B

In a follow-up study C-terminal proSP-B and proSP-B values in serum and bronchoalveolar samples (BAL) of patients diagnosed with idiopathic pulmonary pneumonia were assessed. 50 patients were included in the study. The average follow-up time was approximately one year, and ranging from less than 1 year to about 3 years. Patients who clinically experienced disease progression during that time had higher levels of C-terminal proSP-Band proSP-B at time point 1 compared to those who experienced disease regression or who stayed stable over the course of time. The mean values for the progression group were 2331 ng/mL (C-terminal proSP-B) and 675 ng/mL (proSP-B), of the regression group 1292 ng/mL (C-terminal proSP-B) and 440 ng/mL (proSP-B) while the patient group who remained stable had initial mean values of 1893 ng/mL (C-terminal proSP-B) and 625 ng/mL (proSP-B).C-terminal proSP-B.

Similar pattern as in the serum samples was also observed for BAL samples of the same cohort.

### Example 2b: Short term monitoring

- Patients: IPF (n = 20; prospectively collected)
- serial analysis, 2 different time points at intervals of three months
- Sample types: serum
- Measurements: C-terminal proSP-B, proSP-B

### Results:

C-terminal proSP-B and proSP-B mean and Median results at the two point for IPF:
- No significant difference for mean and median values at the 2 time points for total cohort, but individual changes detectable. Thus, the markers are valuable in individual patients. Changes were, in particular detectable, in patients with severe IPF.
- Correlation with pulmonary function testing: C-terminal proSP-B and proSP-B correlate with DLCO (diffusion capacity for carbon monoxide) and 6 minute-walk testing
- But only very weak correlation with lung function parameter (forced vital capacity) in total cohort,

The following table shows C-terminal proSP-B and proSP-B mean and median results at the two point for IPF

| | C-terminal proSP-B (ng/mL) | | proSP-B (ng/mL) | |
|---|---|---|---|---|
| | Mean | Median | Mean | Median |
| Healthy | 186.8 | 52.2 | 83.8 | 31.8 |
| IPF (visit 1) | 2599.1 | 2503 | 788.4 | 710.5 |
| IPF (visit 2) | 2531.1 | 2482 | 738.4 | 690.6 |

### Example 3: Case studies

### Patient F16

69 years old female patient, ex smoker, BMI 37.3 clinically assessed as disease progression from timepoint 1 to timepoint 2 (approx 1 year)

| | | **Timepoint 1** | | **Timepoint 2** | |
|---|---|---|---|---|---|
| | | | | | |
| **proSP-B results** | C-terminal proSP-B (ng/mL) | 2934 | | 3631 | |
| | proSP-B (ng/mL) | 897,9 | | 847,6 | |
| | | | | | |

| **Lung function testing** | | | | | |
|---|---|---|---|---|---|
| | RV actual value [1] | 1,6 | | 1,2 | |
| | RV normal level % | 70 | | 52 | |
| | TLC actual value [1] | 3,5 | | 2,5 | |
| | TLC normal level % | 62 | decreased | 43 | further decreased |
| | FVC actual value [1] | 1,9 | | 1,3 | |
| | FVC normal level % | 68 | moderate | 46 | severe |
| | IVC actual value [1] | 2 | | 1,3 | |
| | IVC normal level % | 54 | | 35 | |
| | FEV1 actual value [1] | 1,8 | | 1,2 | |
| | FEV1 normal level % | 64 | moderate | 42 | severe |
| | | | | | |
| **Blood gas analysis** | Pa02 [mmHg] | 61 below 11 02 | | 41 below 41 O2 | |
| | PaCO2 [mmHg] | 41 below 11 02 | | 76 below 41 O2 | |
| | | | | | |

| **DLCO/T LCO** | | | | | |
|---|---|---|---|---|---|
| | TLCOc SB actual value [mmol/min/kPa/l] | 3,4 | | not possible | |
| | TLCOc SB normal level % | 45 | moderate severity | | |
| | TLCOc/VA actual value [mmol/min/kPa/l] | 1,1 | | not possible | |
| | TLCOc/VA normal level % | 79 | | | |
| | | | | | |

| **Medication** | | | | | |
|---|---|---|---|---|---|
| | Cortisol therapy | Prednisolon 10mg/d | | Prednisolon 10mg/d | |
| | Immunsuppression | Azathioprin 150 mg/d, Aviptadil | | Aza ab, Cyclophosphamide 5 cycles | |

68 year-old female patient with massive deterioriation of IPF from timepoint 1 to timepoint 2.

Under massive oxygenation (from 11 to 4 1 at time point 2) massive retention of CO2 (76 mmHg at timepoint 2, normal 40 mmHg) and insuffient delivery of 02 (41 mm Hg under 41 oxygen, normal 95 mm Hg) indicating severely impaired gas diffusion.

Severely impaired gas diffusion and gas retention does not allow measurement of diffusion capacity at time point 2 any more due to severe hypoxemia and CO2 retention. Risk of additional CO retention under those conditions if CO diffusion capacity measuremenst would be performed..

Lung function testing may only partially be useful in indicating disease severity and progression as patient is under massive oxygen supply.

Switch in medication between time point 1 and 2 did not prevent massive disease deterioration.

### Conclusion:

C-terminal proSP-B indicates the disease progression and C-terminal proSP-B concentration correlate with disease severity. The higher C-terminal proSP-B concentrations the worse the clinical status of the patient.

C-terminal proSP-B monitored over time can indicate the therapy effectiveness. Although the medication was changed, the disease progressed.

C-terminal proSP-B levels monitored over time may identify fast and slow progressors. C-terminal proSP-B levels significantly increased within one year.

C-terminal proSP-B determinations may be used for therapy monitoring when current gold standard methods (determination of CO diffusion capacity) are not applicable any more due to disease severity. In this respect C-terminal proSP-B levels may also be determined as bedside test. proSP-B may not indicate the same as C-terminal proSP-B as levels almost remained constant, respectively, slightly decreased.

### Patient F48

54 years- old female patient, BMI 25, non smoker; clinically assessed as disease progression from time point 1 to time point 2 (approx 3 years)

| | | **Timepoint 1** | | **Timepoint 2** |
|---|---|---|---|---|
| **C proSP-B and proSP-B results** | | | | |
| | C-terminal proSP-B (ng/mL) | 1638 | | 2593 |
| | proSP-B (ng/mL) | 400 | | 537,6 |
| | | | | |

| **Lung function testing** | | | | |
|---|---|---|---|---|
| | RV actual value [1] | 1,3 | | 0,9 |
| | RV normal level % | 63 | | 41 |
| | TLC actual value [1] | 2,6 | | 2,3 |
| | TLC normal level % | 44 | | 39 |
| | FVC actual value [1] | 1,3 | | 1,5 |
| | FVC normal level % | 39 | | 45 |
| | IVC actual value [1] | 1,3 | | 1,5 |
| | IVC normal level % | 33 | | 37 |
| | FEV1 actual value [1] | 1,1 | | 1,2 |
| | FEV 1 normal level % | 38 | | 39 |

| **Blood gas analysis** | | | | |
|---|---|---|---|---|
| | PaO2 [mmHg] | 60 | | 54 |
| | PaCO2 [mmHg] | 33 | | 40 |
| | | | | |

| **DLCO/TLCO** | | | | |
|---|---|---|---|---|
| | TLCOc SB actual value [mmol/min/kPa/l] | not possible | | not possible |
| | TLCOc SB normal level % | | | |
| | TLCOc/VA actual value [mmol/min/kPa/l] | not possible | | not possible |
| | TLCOc/VA normal level % | | | |
| | | | | |

| **Medication** | | | | |
|---|---|---|---|---|
| | Cortisol therapy | Prednisolon short-time therapy 100 1-0-1 | | Prednisolon extended therapy 7,5 mg/d |
| | Immunsuppression | no | | Z.n. 12 Ebdoxan 750 mg and MMF, Azathioprin 150 mg |

The 54 years old female patient was clinically characterized as IPF progressor over a period of time of approx. 3 years.

The patient is also already in an advanced disease stage at time point 1, as all the lung function test parameters are below normal. However, they remain almost constant over 3 years. Similarly, the arterial blood gas pressure (pa02 and paCO2) are within the same range. However, the CO diffusion capacity measurements, as one of the key methods currently used in IPF monitoring is not possible at both time points (PaCO2 is almost normal at timepoint 2, but Pa02 is reduced).

C-terminal proSP-B and to lower extent proSP-B are raising over this period of time, indicating disease progression. Compared to case F16 the raise took place over a much longer period of time (approx 3 years) indicating that C-terminal proSP-B may be used to identify fast and slow progressors:

C-terminal proSP-B in patient F 48 raised by 955 ng/mL over 3 approx 3 years (318 ng/mL per year) while in patient F 16 the concentration raised by 697 ng/mL in 1 year, e.g. approximately twice as much as in patient F48.

### Patient F24

### 71 years old male patient, BMI 29, non smoker clinically assessed as mildly progressing from timepoint 1 to timepoint 2 (< 1 year)

| | | **Timepoint 1** | **Timepoint 2** |
|---|---|---|---|
| **C-terminal proSP-B and proSP-B results** | | | |
| | C-terminal proSP-B (ng/mL) | 1875 | 1641 |
| | proSP-B (ng/mL) | 651,4 | 558,1 |
| | | | |

| **Lung function testing** | | | |
|---|---|---|---|
| | RV Ist [1] | 2 | 2 |
| | RV normal level % | 83 | 84 |
| | TLC actual value [1] | 5,6 | 5,9 |
| | TLC normal level % | 73 | 77 |
| | FVC actual value [1] | | 3,6 |
| | FVC normal level % | | 83 |
| | IVC actual value [1] | 3,8 | 3,8 |
| | IVC normal level % | 75 | 75 |
| | FEV1 actual value [1] | 2,4 | 2,6 |
| | FEV 1 normal level % | 68 | 76 |
| | | | |

| **Blood gas analysis** | | | |
|---|---|---|---|
| | PaO2 [mmHg] | 74 | 77 |
| | PaCO2 [mmHg] | 39 | 39 |
| | | | |

| **DLCO/TLCO** | | | |
|---|---|---|---|
| | TLCOc SB actual value [mmol/min/kPa/l] | 3,1 | 3,2 |
| | TLCOc SB normal level % | 33 | 35 |
| | TLCOc/VA actual value [mmol/min/kPa/l] | 1,6 | 0,7 |
| | TLCOc/VA normal level % | 50 | 52 |
| | | | |

| **Medication** | | | |
|---|---|---|---|
| | Cortisol therapy | Z.n. systemic Stero-idtherapy 2001 | Z.n. systemic Steroidtherapy 2001 |
| | Immunsuppression | Z.n. Azathioprin, currently Aviptadil | Z.n. Azathioprin, currently Aviptadil |

71-years old male patient who is clinically assessed as mildly progressing within a period of time of less than 1 year.

The patient's lung function as judged by lung function testing, blood gas analysis and diffusion capacity remain almost the same. FEV1 % slightly ameliorates from 68% to 76%, CO diffusion capacity remains almost constant.

C-proSP-N and proSP-B level decrease which is indicative of disease regression.

C-terminal proSP-B and proSP-B may therefore aid in the identification of patients with slow disease progression.

The drug therapy of the patient was unchanged over that period of time.

### Patient F18

### 44 years old female patient, BMI 22, non smoker clinically assessed as active disease from timepoint 1 to timepoint 2 (approx 2 year)

| | | **Timepoint 1** | **Timepoint 2** |
|---|---|---|---|
| **C-terminal proSP-B and proSP-B results** | | | |
| | C-terminal proSP-B (ng/mL) | 1694 | 959,6 |
| | proSP-B (ng/mL) | 521,2 | 480,2 |
| | | | |

| **Lung function testing** | | | |
|---|---|---|---|
| | RV actual value [1] | 0,9 | 1,3 |
| | RV normal level % | 59 | 88 |
| | TLC actual value [1] | 2,4 | 2,6 |
| | TLC normal level % | 49 | 54 |
| | FVC actual value [1] | 1,4 | 1,2 |
| | FVC normal level % | 50 | 42 |
| | IVC actual value [1] | 1,5 | 1,3 |
| | IVC normal level % | 46 | 40 |
| | FEV1 actual value [1] | 1,2 | 1 |
| | FEV 1 normal level % | 45 | 38 |
| | | | |
| | | | |

| **Blood gas analysis** | | | |
|---|---|---|---|
| | PaO2 [mmHg] | 97 | 57 |
| | PaCO2 [mmHg] | 41 | 40 |
| | | | |

| **DLCO/TLCO** | | | |
|---|---|---|---|
| | TLCOc SB actual value [mmol/min/kPa/l] | not available | not available |
| | TLCOc SB normal level % | | |
| | TLCOc/VA actual value [mmol/min/kPa/l] | not available | not available |
| | TLCOc/VA normal level % | | |
| | | | |

| **Medication** | | | |
|---|---|---|---|
| | Cortisol therapy | Prednisolon extended therapy 10mg/d | Prednisolon extended therapy 20 mg |
| | Immunsuppression | Z.n. Rituximab 500 mg 4x, Endoxan short-time therapy, Azathioprin 100/d since 08/08, MMF 2g/d since 01/09 | Z.n. Rituximab 500 mg 4x, Endoxan short-time therapy, Azathioprin 100/d since 08/08, MMF 2g/d since 01/09 |

44 year old female patient who was assessed having an active IPF

The patient's lung function is clearly diminished with FEV1 Soll % of 45 and 38% at time point 1 and 2, and FVC declining from 50% to 42% over approx 2 years. Those values would classify the patient as having severly impaired lung function. PaCO2 are normal, but pa02 is reduced at timepoint 2. The diffusion capacity values are not available (unclear if it was not possible to perform measurments for medically or other reasons). The drug therapy was changed in the patient and doubled from 10 mg to 20 mg Prednisolon.

The C-terminal proSP-B and proSP-B values decreased in that period of time of 2 years by 734 ng/mL (C-terminal proSP-B) and 41 ng/mL (proSP-B).

C-terminal proSP-B and proSP-B may serve as better indicator for the disease progression in situation where clinical assessment on current methods is inconclusive. Patient was clinically assessed as having an active disease, clinical testing showed that disease status was constant and proSP-B levels indicate that situation has improved. Patient could be categorized into the group of slow progressors.

### Patient F141

### 74 years old male patient

Time between timepoint 1 and 2: three months

| | | **Timepoint 1** | **Timepoint 2** |
|---|---|---|---|
| **C-terminal proSP-B and proSP-B results** | | | |
| | C-terminal proSP-B (ng/mL) | 1608 | 1487 |
| | proSP-B (ng/mL) | 347,3 | 423,2 |
| | | | |

| **Lung function testing** | | | |
|---|---|---|---|
| | RV actual value [1] | | |
| | RV normal level % | | |
| | TLC actual value [1] | | |
| | TLC normal level % | | |
| | **FVC actual value [l]** | 1,95 | 1,67 |
| | **FVC normal level %** | 52,6 | 45,2 |
| | IVC actual value [1] | | |
| | IVC normal level % | | |
| | **FEV1 actual value [l]** | 1,84 | 1,43 |
| | FEV1 normal level % | 65,7 | 51,2 |
| | **FEV1/FVC** | 88,78 | 76,35 |
| | | | |

| **Blood gas analysis** | | | |
|---|---|---|---|
| | PO2 [mmHg] (in calm) | 95,8 | 70,6 |
| | PaCO2 [mmHg] | 35,8 | 37,7 |
| | SaO2-Saturation (%) | 98 | 96 |
| | O2 support (L) | 3 | 3 |
| | | | |

| **DLCO/TLCO** | | | |
|---|---|---|---|
| | TLCOc SB actual value [mmol/min/kPa/l] | unknown | unknown |
| | TLCOc SB normal level % | | |
| | TLCOc/VA actual value [mmol/min/kPa/l] | unknown | unkown |
| | TLCOc/VA Soll in % | | |
| | | | |

| **Medication** | | | |
|---|---|---|---|
| | | unknown | unknown |

74 years old patient with diagnosis of IPF who was followed up after 3 months

As assessed by FEV1 and FVC and blood gas (under 31 of 02 at both timepoints), clinical status of patient slightly worsened. C-terminal proSP-B level, however, decreases which may indicate disease regression or at least stability. C-terminal proSP-B level may be used for the short- and midterm monitoring of IPF and ILD patients.

### Patient F142

### 69 years old female patient, non smoker

Time between timepoint 1 and 2: three months

| | | **Timepoint 1** | **Timepoint 2** |
|---|---|---|---|
| **C-terminal proSP-B and proSP-B results** | | | |
| | C-terminal proSP-B (ng/mL) | 5911 | 5951 |
| | proSP-B (ng/mL) | 1039 | 1023 |
| | | | |

| **Lung function testing** | | | |
|---|---|---|---|
| | RV actual value [1] | | |
| | RV normal level % | | |
| | TLC actual value [1] | | |
| | TLC normal level % | | |
| | **FVC actual value [l]** | 1,37 | 1,63 |
| | **FVC normal level %** | 58,2 | 69,1 |
| | IVC actual value [1] | | |
| | IVC normal level % | | |
| | **FEV1 actual value [l]** | 1,36 | 1,57 |
| | **FEV1 normal level %** | 69,6 | 80,2 |
| | **FEV1/FVC** | 88,85 | 96,22 |
| | | | |

| **Blood gas analysis** | | | |
|---|---|---|---|
| | PO2 [mmHg] (in calm) | 74,1 | 86,1 |
| | PaCO2 [mmHg] | 37,4 | 40,9 |
| | SaO2-Saturation (%) | 96 | 97 |
| | O2 support (L) | 3 | 3 |
| | | | |

| **DLCO/TLCO** | | | |
|---|---|---|---|
| | TLCOc SB actual value [mmol/min/kPa/l] | unknown | unknown |
| | TLCOc SB normal level n % | | |
| | TLCOc/VA actual value [mmol/min/kPa/l] | unknown | unkown |
| | TLCOc/VA normal level % | | |
| | | | |

| **Medication** | | | |
|---|---|---|---|
| | | unknown | unknown |

69 years- old female patient, time point for follow up: after 3 months

Although lung function testing parameter classifying the patient as having a medium impaired lung function, and blood gas under 02 delivery of 3 L, seem to be acceptable, C-terminal proSP-B levels indicate a very severe disease which remains constant over the 3 months.

### Patient F148

### 65 years old male patient, non smoker

Time between timepoint 1 and 2: three months

| | | **Timepoint 1** | **Timepoint 2** |
|---|---|---|---|
| **C-terminal proSP-B and proSP-B results** | | | |
| | C-terminal proSP-B (ng/mL) | 3804 | 4384 |
| | proSP-B (ng/mL) | 254,13 | 1672 |
| | | | |

| **Lung function testing** | | | |
|---|---|---|---|
| | RV actual value [1] | | |
| | RV normal level % | | |
| | TLC actual value [1] | | |
| | TLC normal level % | | |
| | **FVC** actual value **[l]** | 1,55 | 1,83 |
| | **FVC normal level %** | 35,8 | 42,5 |
| | IVC actual value [1] | | |
| | IVC normal level % | | |
| | **FEV1 actual value [l]** | 1,44 | 1,55 |
| | **FEV1 normal level %** | 42,9 | 46,6 |
| | **FEV1/FVC** | 81,14 | 83,37 |
| | | | |

| **Blood gas analysis** | | | |
|---|---|---|---|
| | PO2 [mmHg] (in calm) | 50,2 | 84 |
| | PaCO2 [mmHg] | 41,7 | 47,3 |
| | SaO2-Saturation (%) | 90 | 97 |
| | O2 support (L) | 5 | 5 |

| **DLCO/TLCO** | | | |
|---|---|---|---|
| | TLCOc SB actual value [mmol/min/kPa/l] | unknown | unknown |
| | TLCOc SB normal level % | | |
| | TLCOc/VA actual value [mmol/min/kPa/l] | unknown | unkown |
| | TLCOc/VA normal level % | | |
| | | | |

| **Medication** | | | |
|---|---|---|---|
| | | unknown | unknown |

65 years old male patient whose lung function testing and blood gas values (with 5 1 02 support) remain similar over a period of 3 months, but the C-terminal proSP-B levels are indicating a significant raise from 3804 ng/mL to 4384 ng/mL and therefore likely disease deterioration within the 3 months period.

### Example 4: Assays

The biomarkers proSP-B and C-terminal proSP-B were measured as disclosed in Example 1 and 2 of WO 2012/130731.

### Abbreviations:

TLCO: transfer factor CO
DLCO: diffusing capacity of CO german TLCO
TLCO_SB: transfer factor CO determined by single breath method
VA alveolar volume

RV: residual volume
TLC: Total lung capacity
RV: residual volume
FVC: Forced vital capacity
IVC: Inspiratory capacity
FEV1: Forced expiratory volume in 1 second

## Claims

1. A method for monitoring interstitial lung disease (ILD) in a patient having interstitial lung disease, said method comprising
(a) measuring the level of the biomarker C-terminal proSP-B or proSP-B in a first sample from the patient,
(b) measuring the level of the biomarker C-terminal proSP-B or proSP-B in a second sample from the patient that has been obtained after the first sample, and
(c) comparing the level of the biomarker in the second sample to the level of the biomarker in the first sample.

2. The method of claim 1, wherein the interstitial lung disease is idiopathic pulmonary fibrosis (IPF).

3. The method of claims 1 and 2, wherein the sample is a blood, serum or plasma sample.

4. The method of claims 1 and 2, wherein the sample is a bronchoalveolar lavage sample (BAL).

5. The method of any one of claims 1 to 4, wherein an increase of the level in the second sample as compared to the level in the first sample indicates deterioration of ILD, and/or wherein a decrease of the level in the second sample as compared to the level in the first sample indicates amelioration of ILD.

6. The method of any one of claims 1 to 5, wherein a moderate increase of the level of the biomarker in the second sample as compared to the level of the biomarker in the first sample is indicative for a patient who is a slow progressor of ILD, in particular of IPF, and/or wherein a strong increase of the level of the biomarker in the second sample as compared to the level of the biomarker in the first sample is indicative for a patient who is a fast progressor of ILD, in particular of IPF.

7. The method of any one of claims 1 to 6, wherein the second sample has been obtained at least one months, at least three months, or in particular at least 12 months after the first sample.

8. The method of any one of claims 1 to 7, wherein the patient suffers from a severe form of interstitial lung disease, in particular of IPF.

9. The method of claim 8, wherein the patient suffers from a severe form of interstitial lung disease at the time point at which the first sample has been obtained, at the time point at which the second sample has been obtained, or at both time points.

10. The method of claim 8 or 9, wherein the biomarker is C-terminal proSP-B.

11. A method of assessing whether a patient having interstitial lung disease responds to a therapy against interstitial lung disease (ILD), the method comprising
(a) measuring the level of C-terminal proSP-B or proSP-B in a first sample from the patient,
(b) measuring the level of C-terminal proSP-B or proSP-B in a second sample from the patient that has been obtained after the first sample, and
(c) comparing the level of C-terminal proSP-B or proSP-B in the second sample to the level in the first sample.

12. The method of claim 11, wherein the sample is a blood, serum or plasma sample or a BAL sample, in particular wherein the second sample has been obtained at least one month, at least three months, or at least 12 months after the first sample.

13. The method of claims 11 and 12, wherein a decrease of the level of the biomarker in said second sample as compared to said first sample, or an essentially unchanged level in said second sample as compared to the level in said first sample indicates that the patients responds to said therapy, and/or wherein an increase of the level in said second sample as compared to said first sample indicates that the patient does not respond to said therapy.

14. A device adapted for carrying out a method of the invention is provided comprising
a) an analyzer unit comprising a binding agent which specifically binds to the biomarker C-terminal proSP-B or to proSP-B, said unit being adapted for measuring the level of the biomarker in a first and second sample of a patient having ILD, and
b) an analyzer unit for comparing the determined levels in the first and second sample, whereby interstitial lung disease is monitored or whereby it is assessed whether said patient responds to a therapy against interstitial lung disease, and a computer-implemented algorithm for carrying out the comparison.

15. Use of i) the biomarker C-terminal proSP-B or proSP-B and/or of ii) of a binding agent which specifically binds to the biomarker C-terminal proSP-B or proSP-B in a first and a second sample from a patient having interstitial lung disease (ILD) for a) monitoring interstitial lung disease (ILD) in said patient or for b) assessing whether said patient responds to a therapy against interstitial lung disease.
